# EUROPÄISCHE PATENTANMELDUNG

(11) **EP 0 561 166 A1**
(43) Veröffentlichungstag der Anmeldung: **22.09.1993**
(21) Anmeldenummer: 93102526.6
(22) Anmeldetag: 18.02.1993
(51) Int. Cl.: A61K 9/00

(54) **Dosieraerosole enthaltend den Wirkstoff D-18024 und verwandte Strukturen**

(30) Priorität: 17.03.1992 DE 4208545
(71) Anmelder: ASTA Medica Aktiengesellschaft, D-01277 Dresden (DE)
(72) Erfinder: Hettche, Helmut, Dr., W-6057 Dietzenbach (DE); Engel, Jürgen, Prof., W-8755 Alzenau (DE)

(57) **Zusammenfassung**

Es werden Dosieraerosole mit dem Wirkstoff Flezelastin, dem Treibmittel 2H-Heptafluorpropan (TG 227) und/oder 1,1,1,2-Tetrafluorethan (TG 134a) und dem Suspensionsstabilisator Tagat®TO, Tagat®O und Tagat®O2 sowie Verfahren zur Herstellung der Formulierung beschrieben.

## Beschreibung

D-18024 ist ein Phthalazinonderivat mit antiallergischer und antihistaminischer Wirksamkeit, siehe DE-OS 36 34 942.
D-18024 hat folgende Struktur: 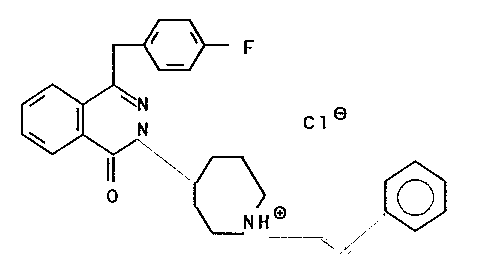
Die zu D-18024 korrespondierende Base trägt den INN (International Nonproprietary Name) Flezelastin. D-18024 kann dem Körper auf verschiedene weise zugeführt werden, beispielsweise auf oralem oder auf inhalativem Weg. Der inhalative Weg ist dem oralen Weg vorzuziehen, da bei inhalativer Anwendung lokal, das heißt in der Lunge, höhere Wirkstoffkonzentrationen erzielbar sind als bei peroraler Anwendung. Die Wirkung setzt schneller ein als nach oraler Gabe. Die zu erwartenden Nebenwirkungen sind schwächer ausgeprägt. Substanzen aus dieser Stoffklasse der Phthalazinonderivate waren bisher wegen ihres stark bitteren Geschmacks nicht inhalativ anwendbar. D-18024 zeigt den stark bitteren Geschmack Uberraschenderweise nicht. Es können auch andere Salze des Flezelastins sowie andere vom Geschmack her akzeptable Phthalazinonderivate eingesetzt werden, gegebenenfalls unter Zusatz von Aromastoffen.

Bisher übliche Dosieraerosole haben den Nachteil, daß sie als Treibmittel chlorierte und fluorierte Kohlenwasserstoffe enthalten, die in Verdacht stehen, am Abbau der Ozonschicht der Erde beteiligt zu sein.

Gegenstand der Erfindung sind Dosieraerosole mit akzeptablem Geschmack, die Flezelastin, oder dessen physiologisch verträglichen Salze, oder weitere Phthalazinone enthalten und diesen Nachteil nicht aufweisen. Als Salzbildner können alle physiologisch unbedenklichen Ionen eingesetzt wrden. Es kommen beispielsweise Anionen starker anorganischer Säuren, wie Chlorid, Sulfat und Phosphat in Frage, weiter Anionen von organischen Säuren, wie Acetat, Gluconat, Malat.

Derartige Dosieraerosole können vom Patienten leicht transportiert und angewendet werden. Sie haben außerdem den Vorteil, daß die Patienten mit der Handhabung bereits vertraut sind. Darüber hinaus ist die Partikelgrößenverteilung in der Aerosolwolke nach Freisetzung aus dem Druckbehälter optimal für einen Transport in die tieferen Abschnitte der Lunge, diejenigen Bereiche, in die der Wirkstoff gelangen muß, damit eine optimale Wirksamkeit erwartet werden kann.

Ein Dosieraerosol von D-18024, dessen Treibgasmischung am Abbau der Ozonschicht der Erde nicht beteiligt ist, kann erhalten werden, indem man D-18024 in 1,1,1,2-Tetrafluorethan (= TG 134a) oder 2H-Heptafluorpropan (=TG 227) oder Mischungen davon suspendiert, gegebenenfalls unter Zusatz weiterer Substanzen wie Ethanol, Pentan, Isopentan, Neopentan, Propan, Butan, i-Butan, Dimethylether, 1,1-Difluorethan, Isopropanol, Aceton, n-Propanol, Propylenglykol, Essigsäureethylester.

Ein Dosieraerosol mit weiteren Phthalazinonderivaten kann mit den gleichen Treibgasen und mit den gleichen Hilfsmitteln hergestellt werden.

Die Suspendierung der Wirkstoffe, zum Beispiel D-18024, kann entweder erfolgen bei normalem Luftdruck, wobei das Suspensionsmedium auf niedrige Temperaturen abgekühlt werden muß (z.B. -35^{o}C bis -55^{o}C) oder innerhalb eines Druckgefäßes, wobei bei Normaltemperaturen (Raumtemperatur 15 bis 25^{o}C) gearbeitet werden kann.

Die Suspendierung von D-18024 wird erleichtert, die Funktion des Dosierventils wird verbessert, und es wird keine Zunahme der Partikelgröße der Formulierung beobachtet durch Zugabe einer oder mehrerer der folgenden Substanzen:
Natürliche Öle wie Maiskeimöl, Olivenöl,
Baumwollsaatöl, Sonnenblumenöl,
Sorbitantrioleat (Span 85),
Sorbitanmonooleat (Span 80),
Sorbitanmonolaurat (Span 20),
Polyoxyethylen (20) sorbitanmonolaurat (Tween 20),
Polyoxyethylen (20) sorbitanmonooleat (Tween 80),
Lecithin (Epikuron 200),
Oleyl Polyoxyethylen(2)ether (Brij 92),
Stearyl Polyoxyethylen(2)ether (Brij 72),
Lauryl Polyoxyethylen(4)ether (Brij 30),
Oleyl Polyoxyethylen(2)ether (Genapol 0-020),
Copolymere aus Ethylenoxid und
Propylenoxid (Pluronics®),
Ölsäure,
Lecithin synthetisch
Diethylenglycoldioleat
Tetrahydrofurfuryloleat
Ethyloleat
Isopropylmyristat
Glyceryltrioleat
Glycerylmonolaurat
Glycerylmonooleat
Glyceryldioleat
Glycerylmonoricinoleat
Propylenglykolmonostearat
Propylenglykoldistearat
Cetylalkohol
Stearylalkohol
Polyethylenglycol 400
Cetylpyridiniumchlorid
Sorbitansesquioleat
Oleylalkohol
Myristylalkohol Polyoxyethylen (25)-glyceryl-trioleat (Tagat®TO)
Polyoxyethylen (30)-glyceryl-monooleat (Tagat®O)
Polyoxyethylen (20)-glyceryl-monooleat (Tagat®O2)
Polyoxyethylenoleat (Atlas G 5507)
Polyoxyethylen (20)-sorbitansesquioleat (Tween®83)
Polyoxyethylen (20)-sorbitantrioleat (Tween®85)
und weitere in TG 227 und/oder TG 134a lösliche Suspensionsstabilisatoren, zum Beispiel lösliche, perfluorierte Suspensionsstabilisatoren wie sie in WO 92/00107 beschrieben werden, so Perfluorbuttersaure, Perfluoroctansäure, Perfluordecansäure und Mischungen aus den Ammoniumsalzen perfluorierter Carbonsäuren, die unter der Bezeichnung FC-143 und FC-95 von 3M in den Handel gebracht werden. Des weiteren können die in WO 92/00061 beschriebenen Suspensionsstabilisatoren in den Formulierungen mit D-18024 und weiteren Phthalazinonderivaten eingesetzt werden, so beispielsweise die Typen Synperonic OP, Synperonic NP, Synperonic LF, Synperonic T, Synperonic T 701, Synperonic T 304, Synperonic T 702, des weiteren die Blockcopolymere von Ethylenoxid und Propylenoxid, die die Handelsnamen Synperonic PE (ICI) oder Pluronic (BASF) tragen. Ferner können die in WO 92/00062 beschriebenen Phosphorsäureester der perfluorierten Sulfonamidoalkohole der allgemeinen Formel 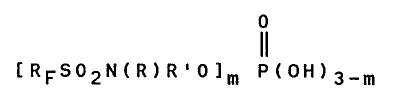
verwendet werden, wobei gilt:
- R_{F}: ist ein perfluorierter Rest der allgemeinen Formel CₙF₂ₙ+1, wobei n die Werte 4 bis etwa 10 annehmen kann, der Rest R_{F} kann auch cycloaliphatisch sein,
- R: kann Wasserstoff oder ein Alkylrest mit 4 bis 12 Kohlenwasserstoffatomen sein,
- R': kann eine Alkylengruppe mit 2 bis 8 Kohlenstoffatomen sein,
- m: ist eine ganze Zahl zwischen 1 und 3.

Des weiteren können die in EP 504 112 beschriebenen Suspensionsstabilisatoren aus der Gruppe der monoacetylierten oder diacetylierten Monoglyceride verwendet werden. Handelsname: Myvacet (Eastman)

Diese Stoffe sind als Zusatzstoffe für verarbeitete Lebensmittel von den Gesundheitsbehörden, zum Beilspiel der FDA in den USA, zugelassen. Desweiteren können die in EP 499 344 beschriebenen Suspensionsstabilisatoren verwendet werden. Darüberhinaus können auch Phospholipide als Suspensionsstabilisatoren eingesetzt werden. Es können auch Gemische aus den unterschiedlichen Suspensionsstabilisatoren eingesetzt werden.

Überraschenderweise wurde nun gefunden, daß bei der Verwendung von Polyoxyethylen (25)-glyceryl-trioleat (Tagat®TO) oder Polyoxyethylen (30)-glyceryl-monooleat (Tagat®O) oder Polyoxyethylen (20)-glyceryl-monooleat (Tagat®O2) eine stabile Suspension der mikronisierten Teilchen der Wirkstoffe mit einem Ethanolzusatz von weniger als 2 Gewichts% (bezogen auf das Aerosolgemisch) erreicht werden kann. Ein so geringer Ethanolgehalt bietet den Vorteil der Unbrennbarkeit der Aerosolmischung und der Stoffwechsel des Patienten wird mit weniger Ethanol als bei bisherigen Formulierungen belastet.

Außerdem ist die Größe der Teilchen der Aerosolwolke geringer, was zu einer erhöhten Deposition der Teilchen in den tieferen Abschnitten der Lunge und damit zu einer erhöhten Wirksamkeit der Zubereitung insgesamt führt. Ein hoher Gehalt an Ethanol hat darüber hinaus einen reduzierten Druck in der Dose zur Folge mit dem Ergebnis, daß die Suspension schlechter versprüht wird. Statt Ethanol kann jedoch auch Isopropanol, Aceton oder Essigsäureethylester eingesetzt werden.

Zur Verminderung der Agglomeration der suspendierten Partikel ist der Zusatz mikronisierter Substanzen wie Natriumsulfat, Natriumchlorid, Saccharose, insbesondere aber Lactose vorteilhaft.
Die Suspension wird homogenisiert und anschließend in Druckdosen abgefüllt, die mit einem Dosierventil verschlossen sind oder anschließend verschlossen werden.

### Beispiel 1

1000 g 2H-Heptafluorpropan (= Teibmittel 227) werden auf eine Temperatur von etwa -55^{o}C abgekühlt und unter Rühren mit einer Lösung aus 11,7 g Ölsäure in 52,5 g absolutem Ethanol versetzt. Anschließend werden 16,8 g mikronisiertes D-18024 und 16,8 g wasserfreie mikronisierte Lactose zugesetzt und die entstandene Suspension intensiv homogenisiert. Die Suspension wird unter weiterem Kühlen und Rühren mit gekühltem Treibmittel 227 auf 1170,0 g aufgefüllt und sodann unter Rühren in Metalldosen abgefüllt, die mit Dosierventilen verschlossen werden, welche pro Hub 50µl der Suspension freisetzen. Pro Hub wird damit 1 mg D-18024 freigesetzt.

### Beispiel 2

Es wird gearbeitet wie in Beispiel 1, jedoch werden statt 11,7 g Ölsäure 11,7 g Polyoxyethylen (25)-glycerol-trioleat und statt 52,5 g absolutem Ethanol 11,7 g absoluter Ethanol eingesetzt.

### Beispiel 3

Es wird gearbeitet wie in Beispiel 1, jedoch wird statt 2 H-Heptafluorpropan eine Mischung aus 4 Gewichtsteilen 2H-Heptafluorpropan und einem Gewichtsteil 1,1,1-2-Tetrafluorethan eingesetzt.

### Beispiel 4

Es wird gearbeite wie in Beispiel 1, jedoch wird statt 2H-Heptafluorpropan eine Mischung aus
13 Gewichtsteilen absolutem Ethanol und
40 Gewichtsteilen 1,1,1,2-Tetrafluorethan
eingesetzt.

### Beispiel 5

Es wird gearbeitet wie in Beispiel 1, jedoch wird statt 11,7 g Ölsäure die gleich Menge Pluronic® F 68 (ein Polyoxyethylenpolyoxypropylen-Polymer, Hersteller: Wyandotte Chemicals Corp., Wyandotte, Michigan, USA) eingesetzt.

## Patentansprüche

1. Pharmazeutische Zusammensetzung aus Flezelastin oder dessen physiologisch verträglichen Salzen, den Treibgasen und dem Suspensionsstabilisator, dadurch gekennzeichnet, daß als Suspensionsstabilisator Polyoxyethylen (25)-glyceryl-trioleat (Tagat®TO) oder Polyoxyethylen (30)-glyceryl-monooleat (Tagat®O) oder Polyoxyethylen (20)-glyceryl-monooleat (Tagat®O2) oder Ölsäure oder Pluronic®F 68 oder perfluorierte Carbonsäuren mit 4 - 10 Kohlenstoffatomen oder Salze der perfluorierten Carbonsäuren oder Synperonic-Typen oder Phosphorsäureester von perfluorierten Sulfonamidoalkoholen oder monoacetyliertes Monoglycerid oder diacetyliertes Monoglycerid oder Phospholipid eingesetzt wird oder Gemische aus vorstehenden Substanzen.

2. Pharmazeutische Zusammensetzung gemäß Anspruch 1, dadurch gekennzeichnet, daß der Gehalt an Suspensionsstabilisator, bezogen auf das Gesamtgewicht der Mischung, zwischen 0,01 und 5 Gewichts% beträgt.

3. Pharmazeutische Zusammensetzung gemäß Anspruch 1, dadurch gekennzeichnet, daß der Gehalt an Suspensionsstabilisator, bezogen auf das Gesamtgewicht der Mischung, zwischen 0,2 und 2,5 Gewichts% liegt.

4. Pharmazeutische Zusammensetzung gemäß Anspruch 1, dadurch gekennzeichnet, daß der Gehalt an Suspensionsstabilisator bezogen auf das Gesamtgewicht der Mischung, zwischen 0,75 und 1,5 Gewichts% beträgt.

5. Verfahren zur Herstellung einer pharmazeutischen Zusammensetzung gemäß Anspruch 1, dadurch gekennzeichnet, daß man ein Treibmittel oder ein Treibmittelgemisch, einen Suspensionsstabilisator, gegebenenfalls Ethanol und den Wirkstoff mischt und in Dosen abfüllt.

6. Verfahren zur Herstellung einer pharmazeutischen Zusammensetzung gemäß Anspruch 5, dadurch gekennzeichnet, daß der Gehalt an Suspensionsstabilisator zwischen 0,01 Gewichts% und 5 Gewichts% liegt.

7. Verfahren zur Herstellung einer pharmazeutischen Zusammensetzung gemäß Anspruch 6, dadurch gekennzeichnet, daß der Gehalt an Suspensionsstabilisator zwischen 0,2 Gewichts% und 2,5 Gewichts% liegt.

8. Verfahren zur Herstellung einer pharmazeutischen Zusammensetzung gemäß Anspruch 7, dadurch gekennzeichnet, daß der Gehalt an Suspensionsstabilisator zwischen 0,75 Gewichts% und 1,5 Gewichts% liegt.

9. Pharmazeutische Zusammensetzung gemäß Anspruch 1 -4,
dadurch gekennzeichnet,
daß als Treibmittel TG 227 und/oder TG 134a verwendet wird.

10. Verfahren zur Herstellung einer pharmazeutischen Zusammmensetzung gemäß Anspruch 5 - 8,
dadurch gekennzeichnet,
daß als Treibmittel TG 227 und/oder TG 134a verwendet wird.
